(19) Europäisches Patentamt
European Patent Office
Office européen des brevets

(11) Publication number: **0 456 229 A2**

# (12) EUROPEAN PATENT APPLICATION

(21) Application number: **91107524.0**

(22) Date of filing: **08.05.91**

(51) Int. Cl.[5]: **C12N 9/16**, C12P 19/60, //A61K47/48,A61K39/395

The microorganism(s) has (have) been deposited with American Type Culture Collection under number(s) 55031.

(30) Priority: **10.05.90 US 523695**

(43) Date of publication of application: **13.11.91 Bulletin 91/46**

(84) Designated Contracting States: **AT BE CH DE DK ES FR GB GR IT LI LU NL SE**

(71) Applicant: **Bristol-Myers Squibb Company**
**345 Park Avenue**
**New York, N.Y. 10154(US)**

(72) Inventor: **Yosuke, Sawade**
**2-15-14-101, Meguro-Honcho**
**Meguro-Ku, Tokyo(JP)**
Inventor: **Tomokazu, Ueki**
**14-11-101 Fujimi-cho**
**Chigasaki-shi, Kanagawa(JP)**
Inventor: **Satoshi, Yamamoto**
**2-14-404, Sakonyama, Asahi-ku**
**Yokohama-shi, Kanagawa(JP)**
Inventor: **Koji, Tomita**
**5-2-3 Kami-yohga**
**Setagawa-ku, Tokyo(JP)**
Inventor: **Yasuo, Fukagawa**
**3-9-2, Imaizumidai**
**Kamakura-shi, Kanagawa(JP)**
Inventor: **Toshikazu, Oki**
**4-20-10, Shod**
**Sakae-ku, Yokohama-shi, Kanagawa(JP)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

## (54) Arylsulfatase.

(57) The present invention relates to an arylsulfatase of microbial origin. The enzyme may be linked to an antibody against a tumor-associated antigen and the resulting conjugate used in conjuction with a sulfated prodrug of a 4'-demethylepipodophyllotoxin glucoside derivative, particularly etoposide in cancer chemotherapy.

EP 0 456 229 A2

## BACKGROUND OF THE INVENTION

The present invention relates to a sulfatase and a method for its production by fermentation. More particularly, it relates to a sulfatase isolated from a species of Streptomyces.

One of the major problems in human cancer chemotherapy is the nonspecific action of antitumor agents which can cause unwanted damage to normal cells. Numerous attempts have been made to more selectively deliver a cytotoxic agent to or near the tumor site thereby minimizing toxicity to normal tissues. A great deal of effort in this area has been devoted to linking a cytotoxic agent to a second component which may have a higher affinity for tumor cells than for normal cells, for example an antibody, a hormone, a lectin, or a polymer.

More recently, a different approach has been proposed which involves administering to a tumor bearing host a prodrug of an antitumor agent, in conjunction with an antibody-enzyme (ab-enz) conjugate [see, e.g., P. D. Senter, et al., European Application 302,473, published February 8, 1989]. The conjugate consists of an enzyme that is capable of converting the prodrug into the active parent compound and a tumor-specific antibody which serves to bring the enzyme to the tumor cell surface where the enzyme would act on the prodrug. This method can thus potentially create a higher concentration of the antitumor drug in the vicinity of the tumor to which the ab-enz conjugate is bound. For use in the ab-enz conjugate/prodrug approach, the enzyme is preferably one that is not normally present in the blood stream in very high concentration in order that the prodrug may remain intact until it encounters the enzyme at the tumor site. The prodrug itself may be considerably less cytotoxic than the parent drug; the cytotoxic drug may be one of the commonly used antitumor agents that is amenable to modification to produce a prodrug which can regenerate the parent drug enzymatically.

Etoposide (Ia) and teniposide (Ib) are two clinically established antitumor drugs belonging to a class of compounds generally known as 4'-demethylepipodophyllotoxin glucosides (DEPG). The general structure of 4'-demethylepipodophyllotoxin glucosides is depicted below as formula (I) wherein $R^1$ may be, for example, $C_{1-10}$alkyl, 2-thienyl, furyl, or phenyl:

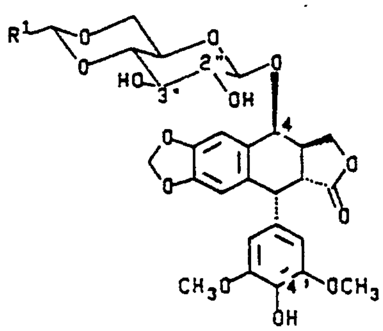

Ia: $R^1$=$CH_3$
Ib: $R^1$=2-thienyl

The hydroxyl groups and phenol group of DEPG may be derivatized to provide a suitable prodrug as substrate for an ab-enz conjugate. In fact, the effectiveness of etoposide 4'-phosphate in combination with a monoclonal antibody-alkaline phosphatase conjugate has been demonstrated in a murine human colon carcinoma xenograft model [Senter, et al., supra].

In addition to etoposide 4'-phosphate, etoposide sulfates are also compounds known in the art. These derivatives are disclosed in Japanese Kokai 88/192,793 and are depicted as formula (II) below:

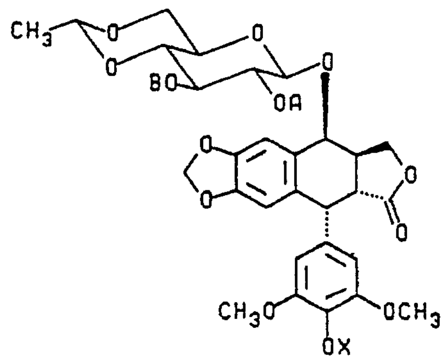

II

wherein one of A, B, and X is the group $-SO_3H$ and the others are H.

Etoposide 4'-sulfate (A = B = H; X = $-SO_3H$) appears to be much less cytotoxic than etoposide itself, requiring a very large dose to achieve the same degree of activity as etoposide. This may indicate that etoposide 4'-sulfate is not facilely converted into etoposide in vivo and, thus, may be more suitably used as a prodrug in combination with an ab-enz conjugate. The enzyme needed to effect the conversion of etoposide 4'-sulfate into etoposide would be a sulfatase which catalyzes the hydrolysis of a sulfate ester to the corresponding hydroxyl compound as follows:

$$R\text{-}O\text{-}SO_3^- + H_2O \xrightarrow{\text{sulfatase}} R\text{-}OH + SO_4^{2-}$$

Several types of sulfatase have so far been studied, and these include Type I and Type II arylsulfatases, steroid sulfatases, glycosulfatases, choline sulfatases, alkylsulfatases, and myrosulfatases. (For a review on sulfatases, see "The Hydrolysis of Sulfate Esters" by A. B. Roy in "The Enzymes", vol. V, pp. 1-19, P. D. Boyer, Ed., Academic Press, 1971.) Among these, arylsulfatases (aryl sulfate sulfohydrolase, EC 3.1.6.1), which catalyzes the above reaction where R is an aromatic ring, have been isolated from various animal tissues, as well as microbial sources, and have been most extensively studied (for a review on arylsulfatases, see "Arylsulfatases" by R. G. Nicholls and A. B. Roy, ibid, pp. 21-41). It is noteworthy that, even though many sulfatases have been reported, few have been purified to homogeneity. For example, arylsulfatase A from rabbit liver has a molecular weight of approximately 70 kD (monomer), forms a dimer at pH 7.4 and tetramer at pH 4.8, and has been purified 10,000 fold (G. D. Lee and R. L. Van Etten; Arch. Biochem. Biophys., 166, 280-294, 1975); arylsulfatase isolated from ox liver is a glycoprotein having a molecular weight of 107 kD (monomer) (L. W. Nichol and A. B. Roy; J. Biochem., 55, 643-651, 1964 and E. R. B. Graham and A. B. Roy; Biochim. Biophys. Acta, 329, 88-92, 1973).

Arylsulfatase activity in releasing sulfate from lignosulfonate was demonstrated in cell free extracts of Streptomyces sp. L.; however, the enzyme itself was not purified or characterized (Y. L. Steinitz, Eur. J. Appl. Microb. Biotechnol., 13, 216-221, 1981).

Arylsulfatases isolated from various sources are available commercially. These enzymes have been evaluated using etoposide 4'-sulfate as the substrate; however, most of these showed either no or little hydrolytic activity against this compound. Furthermore, none of the commercially available sulfatases are homogeneous and some have unfavorable characteristics such as high molecular weight, low optimum pH, etc., rendering them unsuitable for clinical use.

Against this background, a program was initiated to screen for microbial arylsulfatases which are capable of hydrolyzing a 4'-demethylepipodophyllotoxin glucoside 4'-sulfate to the corresponding 4'-demethylepipodophyllotoxin glucoside. One arylsulfatase, designated Es-2, discovered through this effort is the subject of our copending application U.S. Serial No. 242,376 filed October 20, 1989. Another arylsulfatase, designated Es-1, is the subject of the invention disclosed and claimed herein.

## BRIEF DESCRIPTION OF THE DRAWINGS

Figure 1 shows the activity and stability profile of Es-1 sulfatase as a function of pH.

Figure 2 shows the activity and stability profile of Es-1 sulfatase as a function of temperature.

Figure 3 shows the recovery of enzyme activity of EDTA-treated Es-1 sulfatase by addition of $Ca^{2+}$.

## SUMMARY OF THE INVENTION

The present invention provides a sulfatase capable of catalyzing the conversion of a 4'-demethylepipodophyllotoxin glucoside 4'-sulfate into the corresponding 4'-demethylepipodophyllotoxin glucoside, said sulfatase having a molecular weight of about 45 kD and an isoelectric point of about 4.95.

A further aspect of the present invention provides a method of producing the sulfatase which comprises aerobically cultivating Streptomyces griseorubiginosus S980-14 in a medium containing an assimilable source of carbon and nitrogen until a recoverable amount of the enzyme has been formed, and recovering said sulfatase.

Yet another aspect of the present invention provides a sulfatase producing microorganism Streptomyces griseorubiginosus S980-14 or a mutant thereof, or a variant thereof.

## DETAILED DESCRIPTION OF THE INVENTION

The present invention provides a sulfatase designated herein as Es-1 isolated from an actinomycete, specifically Streptomyces griseorubiginosus S980-14; said sulfatase has a molecular weight of about 45 kD as determined by SDS-PAGE and gel filtration methods and an isoelectric point of about 4.95. The enzyme is capable of catalyzing the conversion of a 4'-demethylepipodophyllotoxin glucoside 4'-sulfate into a 4'-demethylepipodophyllotoxin glucoside as depicted below:

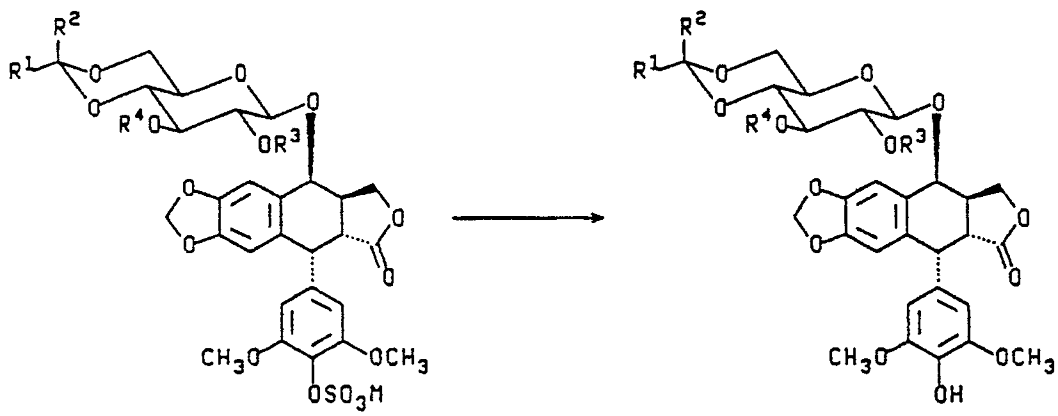

wherein $R^1$ and $R^2$ are each $(C_{1-10})$alkyl, or $R^2$ is H and $R^1$ is selected from the group consisting of $C_{1-10}$alkyl, furyl, thienyl, and phenyl; $R^3$ and $R^4$ are each independently H or an acyl group; and M is H or an alkali metal ion. Acyl group may include, but is not limited to, formyl, acetyl, and benzoyl. Alkali metal ion is, for example, lithium, sodium, and potassium. More particularly, the substrate for Es-1 is etoposide 4'-sulfate or an acylated derivative thereof.

As used herein, "substantially purified" enzyme represents the enzyme showing a single band upon SDS-polyacrylamide gel electrophoresis.

### I. Producing Organism

The enzyme of the present invention is produced by a strain of Streptomyces griseorubiginosus designated as strain S5980-14. This organism was isolated from a soil sample collected in Hanamaki Hot Spring, Iwate Prefecture, Japan. A biologically pure culture of this microorganism has been deposited with the American Type Culture Collection, Rockville, MD, under the accession number ATCC 55031.

(a) Morphology. Aerial mycelium is abundantly formed on the long, branched and non-fragmentary substrate mycelium. Long straight spore-chains (20 to 50 spores per chain) are born monopodially on the aerial hyphae. The spore-chains are formed abundantly in ISP media Nos. 4 and 5, but they are either not formed or scantly formed in ISP media Nos. 2 and 3. The spores are oblong (0.5-0.7 x 0.8-1.2 μm) and have a smooth surface. Motile spores, verticils, or sclerotia are not observed.

(b) Cultural Characteristics. Strain S980-14 shows good or moderate growth on ordinary media. The

aerial mycelium is white and turns light gray, yellowish gray, or reddish gray after sporulation. The reverse side color is dark reddish brown or dark grayish reddish brown. Melanin is produced in ISP media Nos. 1, 6, and 7. The cultural characteristics are shown in Table I.

Table I. Cultural Characteristics of Strain S980-14

| Medium | Growth | Aerial Mycelium | Substrate Mycelium | Diffusible Pigments |
|---|---|---|---|---|
| Sucrose-nitrate agar (Czapek-Dox agar) | Good | Scant; dark orange yellow (72) | Dark reddish brown (44) | Dark brown (59) |
| Tryptone-yeast extract broth (ISP No. 1) | Moderate, floccose, not turbid | None | Colorless | Dark yellowish brown (78) |
| Yeast extract-malt extract agar (ISP No. 2) | Moderate | Poor; white to light gray (264) | Dark yellowish brown (78) | Deep yellowish brown (75) |
| Oatmeal agar (ISP No. 3) | Good | Good; yellowish gray (93) | Dark grayish yellowish brown (81) | Dark yellowish brown (78) |
| Inorganic salts-starch agar (ISP No. 4) | Moderate | Moderate; reddish gray (22) | Dark grayish red (20) | None |
| Glycerol-asparagine agar (ISP No. 5) | Good | Good; yellowish gray (93) | Dark brown (59) | Dark yellowish brown (78) |
| Peptone-yeast extract-iron agar (ISP No. 6) | Moderate | None | Light grayish yellowish brown (79) | Dark brown (59) |
| Tyrosine agar (ISP No. 7) | Good | Good; pale yellow (89) to yellowish gray (93) | Brownish black (65) | Brownish black (65) |
| Glucose-asparagine agar | Moderate | Moderate; light gray (264) | Dark grayish red (20) | None |
| Nutrient agar | Poor | None | Colorless | Moderate yellowish brown (77) |

Observation after incubation at 28°C for 3 weeks. Color name used: ISCC-NBS color-name charts.

(c) Physiological Characteristics. Strain S980-14 hydrolyzes gelatin and starch and produces tyrosinase but not nitrate reductase. NaCl tolerance is seen at 6% but not at 8%. All of the diagnostic sugars (D-glucose, D-xylose, L-arabinose, L-rhamnose, D-fructose, D-galactose, raffinose, D-mannitol, i-inositol, salicine, and sucrose) are utilized for growth. The physiological characteristics of strain S980-14 are shown in Table II.

Table II. Physiological Characteristics of Strain S980-14

| Hydrolysis of: | | Utilization of:* | |
|---|---|---|---|
| Gelatin | + | Glycerol | + |
| Starch | + | D-Arabinose | $+^{W}$ |
| | | L-Arabinose | + |
| Milk coagulation | - | D-Xylose | + |
| Peptonization | - | D-Ribose | + |
| | | L-Rhamnose | + |
| Production of: | | D-Glucose | + |
| | | D-Galactose | + |
| Nitrate reductase | - | D-Fructose | + |
| Tyrosinase | + | D-Mannose | + |
| | | L-Sorbose | - |
| Tolerance to: | | Sucrose | + |
| | | Lactose | + |
| Lysozyme, 0.001% | + | Cellobiose | + |
| 0.01% | - | Melibiose | + |
| NaCl, 1%-6% | + | Trehalose | + |
| 8% or More | - | Raffinose | + |
| pH, 4.5-10.5 | + | D-Melezitose | - |
| | | Soluble Starch | + |
| Temperature: | | Cellulose | - |
| | | Dulcitol | - |
| Growth range | 14°C-43°C | Inositol | + |
| Optimal growth | 30°C-35°C | D-Mannitol | + |
| No growth | 11°C and 46°C | D-Sorbitol | - |
| | | Salicin | $+^{W}$ |

*Basal medium: Pridham-Gottlieb's inorganic medium (ISP No. 9).

w: Weak Utilization

(d) Cell Chemistry. Whole cell hydrolysate contains LL-isomer of diaminopimelic acid. Phospholipids contain phosphatidylethanolamine, phosphatidylglycerol, and phosphatidylinositol. Therefore, strain S980-14 belongs to cell wall Type I and phospholipid Type P-II.

Based on the above-mentioned major characteristics, strain S980-14 was placed in the genus Streptomyces. According to the classificatory keys of Streptomyces by Pridham and Tresner [Genus Streptomyces Waksman and Henrici, pp. 748-829, in R. E. Buchanan and N. E. Gibbons (Ed.) Bergey's Manual of Determinative Bacteriology, 8th Ed., 1974, Williams & Wilkins Co., publ.], the strain is assigned to the gray (GY), Rectus-Flexibilis (RF), chromogenic (C+), and smooth (SM). Among 22 known species of this species group, strain S980-14 is similar in its chromogenicity and sugar utilization profile to S. cacaoi subsp. asoensis IPCR 20-52, S. fulvoviolaceus ATCC 15862, S. griseorubiginosus INA-7712, and S. phaeopurpureus ATCC 23946. Based on the further comparative considerations among strain S980-14 and these four species, the strain was classified as a strain of Streptomyces griseorubiginosus.

II. Enzyme Production

The arylsulfatase Es-1 of the present invention is produced by cultivating Streptomyces griseorubiginosus strain S980-14 or a mutant thereof under submerged conditions in an aqueous nutrient medium. The producing organism is grown in a nutrient medium containing an assimilable carbon source, for example an assimilable carbohydrate. Example of suitable carbon sources include cerelose, fructose, soluble starch, and glycerol. The nutrient medium should also contain an assimilable nitrogen source such as fish meal, yeast extract, or ammonium salts. Inorganic salts such as sodium chloride, potassium chloride, magnesium sulfate, calcium carbonate, phosphates, etc., and trace elements such as copper, manganese,

iron, zinc, etc., are added to the medium if necessary, or they may be present as impurities of other constituents of the medium. The incubation temperature may be any temperature at which the producing strain is able to grow and produce the desired product, e.g., from about 18° C to about 39° C, but it is preferable to conduct the fermentation at about 25° C-35° C, most preferably at about 25° C-30° C. A near neutral pH is preferably employed in the medium, and production of the enzyme is generally carried out for a period of about 4 to 8 days. Ordinarily, optimum production is achieved in about 5-6 days. For preparation of relatively small amounts of the enzyme, shake flask or surface culture can be employed, but for the preparation of larger amounts, submerged aerobic culture in sterile tanks is preferred. When tank fermentation is to be carried out, it is desirable to produce a vegetative inoculum in a nutrient broth by inoculating the broth with spores from the organism and, when a young active vegetative inoculum has been obtained, transferring the inoculum aseptically to the fermentation tank medium. Further agitation may be provided with a mechanical impeller. Antifoam agents, such as lard oil or silicone oil, may also be added if needed.

It is to be understood that the present invention is not limited to the use of the particular preferred Streptomyces griseorubiginosus strain S980-14 described above or to organisms fully answering the above description. It is especially intended to include other strains or mutants of the said organism which can be produced by conventional means, such as x-rays radiation, ultraviolet radiation, treatment with nitrogen mustards, phage exposure, and the like, and which retain the ability to produce the arylsulfatase Es-1 of the present invention.

III. Isolation and Purification of Enzyme

The arylsulfatase of the present invention may be isolated from the fermentation broth using conventional protein separation methodologies such as dialysis, ultrafiltration, gel filtration, isoelectric precipitation, salting out, electrophoresis, ion-exchange chromatography, and affinity chromatography. A combination of these techniques in sequence is generally used to purify the protein to apparent homogeneity. Protein purification is preferably carried out at reduced temperature, preferably at about 0°-5° C. The isolation and purification process may be monitored and guided by enzyme activity assay using p-nitrophenylsulfate or other suitable arylsulfates as substrate or by physical methods such as UV or HPLC techniques. A typical isolation purification sequence is provided below for illustrative purpose only, and it will be appreciated by those skilled in the art that different sequences using other methods may also be used so long as the protein is obtained in high purity and retains its biological activities.

The fermentation broth of Streptomyces griseorubiginosus S980-14 is filtered to remove the insoluble mass, and the volume of the filtrate is reduced at room temperature using ultrafiltration. The concentrated solution is treated with ammonium sulfate to achieve about 30% (w/v) saturation. The resulting solution is allowed to stand overnight at about 4° C, and then centrifuged to remove the non-sulfatase protein. The supernatant containing Es-1 sulfatase is subjected to purification by gee affinity column chromatography using as adsorbant a hydrophobic polyvinyl polymer such as Butyl-Toyopearl 650 C (a butylated polyvinyl alcohol polymer carrier marketed by Tosoh Co. Ltd.). The column is washed with 30% ammonium sulfate in a suitable buffer such as 50 mM Tris-HCl at pH 7.5, and then eluted with the same buffer. Fractions containing sulfatase activity are combined and further purified by anion-exchange chromatogragphy using a suitable anion-exchanger such as DEAE-Cellulose. The elution buffer is suitably Tris-HCl at about pH 7.5 used in linear concentration gradient from 50 mM to 300 mM. The combined active fractions are again chromatographed on a hydrophobic gel affinity column of butylated polyvinyl alcohol sorbent such as Butyl-Toyopearl 650 μ (having a smaller size than Butyl-Toyopearl 650 C) using a linear concentration gradient of ammonium sulfate from 30% to 10% in 50 mM Tris-HCl buffer at pH of about 7.5. Active fractions are pooled and purified by chromatofocusing on a column of Polybuffer Exchanger 94 (Pharmacia LKB) using Polybuffer 74 (Pharmacia LKB) at about pH 4.0 as eluant. Active fractions are combined and further purified on a column of cross-linked cellulose such as Cellulofine GC-700-m (Seikagaka Kogyo, Co.) using 250 mM Tris-HCl, pH 7.5 as eluant; the pooled active fractions are concentrated by ultrafiltration to provide the sulfatase of the present invention.

Sulfatase activity during purification is monitored by determining spectrophotometrically the conversion of p-nitrophenylsulfate into p-nitrophenol. Specifically, the following assay method is used. 50 μl of a p-nitrophenylsulfate solution (1 mg/ml in 50 mM Tris-HCl, pH 7.5), 50 μl of a sample, and 100 μl of 50 mM Tris-HCl, pH 7.5, are mixed and incubated for 30 minutes at 37° C. If the activity is weak, the incubation time is prolonged up to 18 hours. The liberated p-nitrophenol is determined spectrophotometrically at 415 nm. From standard curves of p-nitrophenol and protein concentrations, the unit (U) of sulfatase activity and the specific activity of sulfatase per mg of protein are calculated. A unit of sulfatase activity is defined as the amount of enzyme which can hydrolyze one nanomole of p-nitrophenyl sulfate to p-nitrophenol in one

minute at 37° C at pH 7.5; protein concentration is determined by measuring the absorption at 280 nm throughout purification and, in case of homogeneous protein, by the Bradford method [Bradford, M et al., Anal. Biochem., 1976, 72:248-254] using bovine serum albumin as the standard protein.

IV. Characterization of Sulfatase Es-1

Various physical, chemical, and biochemical properties of sulfatase Es-1 have been determined, and the results are reported below. For comparison purposes, the corresponding values for sulfatase Es-2 which were determined using similar procedures and under similar conditions are also provided. Experimental details for Es-2 are reported in our copending application U.S. Serial No. 424,376 filed October 20, 1989.

A. Molecular Weight (MW) Determination.

(a) By Gel Filtration. Purified Es-1 sulfatase was chromatographed on TSK-GEL (TOYOPEARL, HW-55F, TOSOH, Φ 2.5 x 70 cm: Vt, developing solvent: 250 mM Tris-HCl, pH 7.5) along with standard proteins, horse myoglobin (MW: 17,800), chymotrypsinogen (MW:25,000), egg albumin (MW: 45,000), bovine serum albumin (MW: 67,000), and blue dextran. Eluate was fractionated in 1 ml fractions. Blue dextran was eluted at fr. no. 110 (Vo), chymotrypsinogen at fr. no. 188 (Ve), egg albumin at fr. no. 173 (Ve), bovine serum albumin at fr. no. 168 (Ve), and Es-1 at fr. no. 171 (Ve). $K_{av}$ was calculated by equation (1), and the MW of Es-1 was read from plotting $K_{av}$ against MW. By this method the molecular weight of Es-1 was determined to be about 45 kD. [MW of Es-2 by gel filtration is about 45 kD.]

$$K_{av} = (Ve - Vo) \div (Vt - Vo) \qquad (1)$$

(b) By SDS-PAGE. A mixture of the sulfatase (25 μg in Tris-HCl (5OmM, 40 μl, pH 7.5), 10% sodium dodecylsulfate (SDS, 10 μl), and 50% glycerin (5 μl, containing 0.05% bromophenol blue) was heated at 98° C for one minute. The reaction mixture was applied into a well of SDS-polyacrylamide gel (12.5%). Electrophoresis was carried out in a solution of Tris-HCl 0.31%, glycine 1.44%, and SDS 1.0% at 200 - 300 V (floating) and 40 mA (constant) for 2 hours at 25° C. The gel was stained with 0.25% Coomassie Brilliant Blue R250 and washed with 7% acetic acid. The molecular weight of the sulfatase was determined to be about 45 kD relative to marker proteins of known molecular weights.

2-Mercaptoethanol treated Es-1 was subjected to SDS-PAGE and the result showed only one protein band showing the same mobility as the 2-mercaptoethanol untreated protein preparation. This indicates that Es-1 exists as a monomer.

B. Isoelectric Point Determination.

Isoelectric point was estimated by column chromatography using Chromatofocusing gel PBE94 (Pharmacia-LKB Biotechnology). The protein was applied on top of the gel column which had been previously equilibrated with 25 mM imidazole-HCl, pH 7.4, and eluted with Polybuffer 74-HCl, pH 4.0. The pH and absorption at 280 nm of each fraction were measured. The isoelectric point of the sulfatase was determined to be about 4.95 using this method. [Isoelectric point of Es-2 is 5.6.]

C. Enzyme Activity Profile.

(a) Optimum pH. The stock enzyme solution (0.6 u in 50 μl of 50 mM Tris-HCl, pH 7.5) was added to 100 μl each of the following buffers: 500 mM sodium acetate (pH 4.0-5.5), 500 mM Tris-maleate (pH 5.5-7.5), 500 mM Tris-HCl (pH 7.5-9.0), and 500 mM glycine-NaoH (pH 9.0-10.0). The substrate, p-nitrophenylsulfate, was dissolved in each of the above buffers (1 mg/ml). The enzyme-buffer solution (150 μl) was mixed with 50 μl of the substrate solution in the same buffer as the enzyme, and the mixture was incubated for 30 minutes at 37° C. The reaction was quenched by addition of 100 μl of 0.2 N sodium hydroxide solution. The enzyme activity in each buffer was determined spectrophotometrically by UV at 415 nm. The optimal pH of the enzyme was thus determined to be about 8.5 (Fig. 1A). [Optimum pH for Es-2 is about 9.0.]

(b) pH Stability. The stock enzyme solution was dialyzed against water to remove salts. The dialyzed solution (10 μl) was mixed with 40 μl of 50 mM Tris-HCl buffer (pH 7.5). An aliquot (10 μl) of this solution was mixed with 40 μl each of the buffer listed in (a) and incubated for 30 minutes at 30° C. To

this solution (50 µl) were added 100 µl of 500 mM Tris-HCl (pH 9.0) and 50 µl of substrate in water (1 mg/ml), and the mixture was incubated at 37°C for 30 minutes to determine the residual enzyme activity. The enzyme showed maximum stability at pH of around 8 to 9 (Fig. 1B). [Es-2 maximum stability is at about pH 8.5.]

(c) Optimum Temperature. Enzyme (0.6 u) in 150 µl of 50 mM Tris-HCl (pH 8.5) was incubated with p-nitrophenylsulfate in the same buffer (1 mg/ml, 50 µl) for 30 minutes at various temperatures. The optimal temperature for enzyme activity was shown to be about 37°C (Fig. 2A). [Optimum temperature for Es-2 is about 30°C.]

(d) Temperature Stability. Enzyme preparations (0.6 u) in 50 mM Tris-HCl (150 µl, pH 8.5) were incubated for 15 minutes at various temperatures and then cooled in an ice bath. p-Nitrophenylsulfate in the same buffer (1 mg/ml, 50 µl) was added to the enzyme solution, and the mixture was incubated for 30 minutes at 37°C. The enzyme was stable below 25°C at pH 8.5 and rather stable around 37°C (Fig. 2B). [Es-2 is stable below 30°C at pH 9.0.]

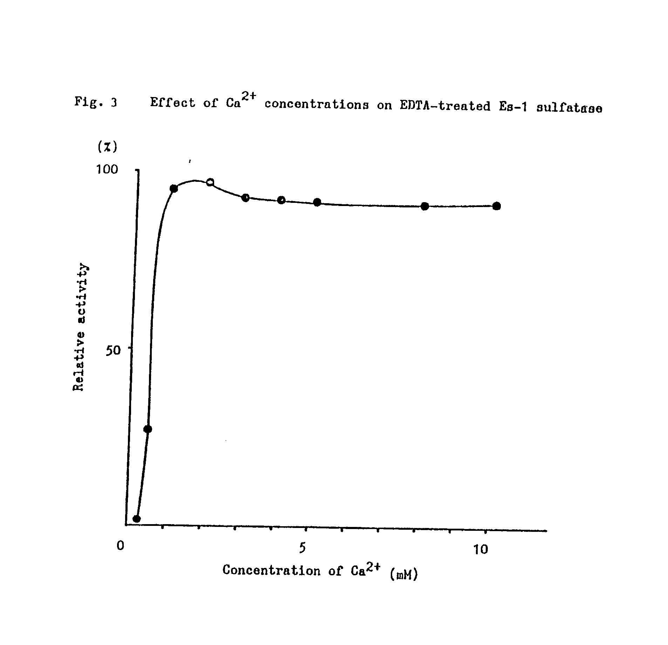

(e) Effect of Ions. Fifty µl of enzyme solution (0.6 u in 50 mM Tris-HCl, pH 8.5), 80 µl of 50 mM Tris-HCl (pH 8.5), 50 µl of p-nitrophenyl sulfate (1 mg/ml in same buffer), and 20 µl of ion (10 mM) were mixed. The solution was incubated for 30 minutes at 37°C. p-Nitrophenol released was spectrophotometrically assayed by UV at 415 nm. The effect of metal ions on enzyme activity is given in Table IV. When the enzyme was treated with 10 mM of EDTA followed by dialysis against water at 4°C, the sulfatase activity disappeared. Addition of 1 mM of $Ca^{++}$ to the EDTA treated enzyme restored 95% of the sulfatase activity, and calcium ion concentrations over 1 mM resulted in sulfatase activity level close to the original level (Fig. 3).

Table IV. Effects of Metal Ions on Sulfatase Activity

| Metal Ion | Relative Activity (%) | Inhibition (%) |
|---|---|---|
| None | 100.0 (100)* | 0 (0) |
| $CoCl_2$ | 57.0 (34) | 43.0 (66) |
| $NiCl_2$ | 66.5 (59) | 33.5 (41) |
| $ZnCl_2$ | 38.7 (18) | 61.6 (82) |
| $BaCl_2$ | 78.7 (83) | 21.3 (17) |
| $CuCl_2$ | 52.7 (56) | 47.3 (44) |
| $MnCl_2$ | 36.5 (34) | 63.5 (66) |
| $FeCl_2$ | 52.5 (12) | 47.5 (88) |
| $CaCl_2$ | 100.9 (213) | 0 (-) |
| $AlCl_3$ | 56.6 (67) | 43.4 (33) |
| $FeCl_3$ | 48.9 (51) | 51.1 (49) |

*Numbers in parenthesis represent values for sulfatase Es-2.

(f) Effect of Enzyme Inhibitors. The protocol used in (e) was followed with the exception that 1 mM of an enzyme inhibitor was used instead of a metal ion. The results are given below in Table V.

Table V. Effect of Enzyme Inhibitors on Sulfatase Activity

| Inhibitor | Inhibition % |
|---|---|
| EDTA | 83.4 (69)* |
| 1,10-Phenanthroline | 50.7 (60) |
| Cysteine | 41.0 (18) |
| Citrate | 72.1 (87) |
| p-Chloromercuribenzoic acid | 69.1 (78) |
| N-Ethylmaleimide | 43.8 (35) |
| Iodoacetic acid | 100.0 (96) |
| 2-Mercaptoethanol | 3.7 (22) |
| Dithiothreitol | 36.8 (34) |
| Imidazole | 0 (14) |
| Phenylmethanesulfonyl fluoride | 21.8 (81) |
| $HgCl_2$ | 100.0 (100) |

*Numbers in parenthesis represent values for sulfatase Es-2.

(g) Effect of Salts. The protocol used in (e) was followed, except that 5 mM of a salt was used instead of 10 mM of a metal ion. The sulfatase activity was strongly inhibited by phosphate ion and sodium chloride. The results are shown in Table VI.

Table VI. Effect of Salts on Sulfatase Activity

| Salt | Inhibition % |
|---|---|
| None | 0 (0)* |
| $K_2HPO_4$ | 70.0 (100) |
| NaCl | 77.7 (58) |
| KCl | 41.4 (36) |
| $(NH_4)_2SO_4$ | 62.8 (44) |
| $K_2SO_4$ | 64.8 (36) |
| $K_2SO_3$ | 47.6 (64) |

*Numbers in parenthesis represent values for sulfatase Es-2.

(h) Substrate Specificity.

(i) Hydrolysis of Etoposide Derivatives. Four etoposide derivatives were tested as substrate for the sulfatase of the invention. The substrates are: etoposide 4'-sulfate (III), 2",3"-di-O-acetyl-etoposide-4'-sulfate (IV), etoposide 2"-sulfate (V), and etoposide 3"-sulfate (VI). The substrate (50 µl, 1 mg/ml in $H_2O$-MeOH 1:1 v/v), the enzyme (50 µl, 0.6 u, 50 mM Tris-HCl, pH 7.5), and 100 µl of 50 mM Tris-HCl, pH 7.5 were mixed and incubated at 37°C for 18 hours. After 18 hours, 100 µl of methanol was added, and 100 µl of sample was analyzed on a silica gel TLC plate using a solvent system consisting of chloroform-methanol (10:1 v/v). The results indicate that, under these test conditions, compounds (III) and (IV) are substrates for the enzyme whereas compounds (V) and (VI) are not.

The substrates, compounds (III), (V), and (VI), are known compounds described in Japan Kokai 88/192,793 and our co-pending U.S. Patent Application USSN 264,940, filed October 31, 1988; the portions of the disclosure of USSN 264,940 relating to the preparation of compounds (III), (V), and (VI) are hereby incorporated by reference. Compound (IV) was prepared according to the following procedure:

To a solution of 2",3"-di-O-acetyletoposide (170 mg, 0.25 mmol) in pyridine (5ml) were added dimethylaminopyridine (DMAP, 3 mg, 0.025 mmol) and sulfur trioxide-pyridine complex (199 mg, 1.25 mmol), and the mixture was stirred for 3 days at room temperature. To this mixture were added further DMAP (3 mg, 0.0025 mmol) and sulfur trioxide-pyridine complex (80 mg, 0.5 mmol), and the mixture was stirred at room temperature for 1 day. An additional amount of sulfur trioxide-pyridine complex (199 mg, 1.25 mmol) was added, and the mixture was stirred for an additional 2-days. The reaction mixture was concentrated in vacuo below 40° C to give a crude solid showing two spots on a silica gel TLC plate (Rf 0.9 and 0.2, methylene chloride:methanol = 5:1). The crude mixture was subjected to silica gel column chromatography (methylene chloride:methanol = 5:1) to provide the starting material (51 mg, Rf 0.9) and the desired sulfate (115 mg, 60%, Rf 0.2). This sulfate (40 mg, 0.054 mmol) was dissolved in a solution of sodium bicarbonate (4.5 mg, 0.054 mmol) in water (4 ml), and the solution was lyophilized to give sodium 2",3"-di-O-acetyletoposide-4'-sulfate (41 mg) as a colorless powder. MP 201° C-230° C. IR $\nu$max (KBr) $cm^{-1}$ 3350 (br), 1750, 1600.

2",3"-Di-O-acetyletoposide used above was prepared according to the procedure provided in our co-pending application USSN 362,555, filed June 7, 1989; that portion disclosing the preparation of this compound is hereby incorporated by reference.

(ii) Hydrolysis of BU-3285T. BU-3285T (0.4 mg/50 µl of $H_2O$), Es-1 sulfatase (0.6 u, 100 µl of 50 mM Tris-HCl, pH 8.5), and 350 µl of 50 mM Tris-HCl, pH 8.5, were mixed and incubated for 18 hours at 37° C. The reaction mixture (20 µl) was spotted on a silica gel plate (silica gel 60 $F_{254}$, Merck Co.), and the plate was developed with a solvent system of $CHCl_3$-MeOH (5:1 v/v). The TLC plate was scanned by UV densitometry at 254 nm. The result indicates that Es-1 sulfatase catalyzes the conversion of approximately 70% BU-3285T to its desulfated derivative.

BU-3285T is disclosed in our co-pending application USSN 431,423, filed November 3, 1989, and the portion relating to the production of the antibiotic is hereby incorporated by reference.

It is clear from the foregoing description that the enzyme Es-1 of the present invention may be distinguished from Es-2 based upon physical, chemical, and biochemical properties. Es-1 thus represents a novel sulfatase produced by a species of Streptomyces.

The characteristics of the arylsulfatase of the present invention render it particularly suitable for clinical use in cancer chemotherapy in which it can be delivered to the vicinity of the tumor where it acts to convert a relatively non-cytotoxic 4'-demethylepipodophyllotoxin glucoside 4'-sulfate into its more cytotoxic parent form. One means of bringing the enzyme close to the tumor is to link the enzyme to an antibody directed to a tumor-associated antigen. This can be accomplished by employing techniques commonly practiced in the art, such as the use of heterobifunctional linkers, examples of which include, but not limited to, N-maleimidobenzoyl succinimide ester, N-succinimidyl-3-(2-pyridyldithio)propionate, and succinimidyl 4-(N-maleimidomethyl)cyclohexane-1-carboxylate. This method is illustrated in Senter, et al., supra. The teachings of EP-A- 302,473 is incorporated herein by reference.

The ab-enz conjugate and the prodrug may be administered by any conventional route of administration, such as intravenous, intramuscular, intraarterial, oral, intralymphatic, intraperitoneal, and intratumoral. Intravenous is the preferred route. The conjugate and prodrug may be given contemporaneously, but preferably, the conjugate is administered prior to the introduction of the prodrug into the host. Optimal dosages and treatment schedules for a given host will, of course, depend on the prodrug and conjugate chosen and will vary according to the particular composition formulated, the route of administration, and the particular situs, host, and disease being treated. Many factors that modify the action of the therapeutic agents will be taken into account, including age, weight, sex, diet, time of administration, route of administration, rate of excretion, condition of the patient, drug combinations, reaction sensitivities, and severity of the disease.

Although the primary use for the enzyme of the present invention is contemplated as being linked to an antibody and the resultant ab-enz conjugate used in conjunction with a prodrug in the therapy of cancer, other uses for the sulfatase are also envisaged. For example, the sulfatase of the present invention is useful for the degradation of lignosulfonate, a water pollutant generated by the pulp and paper industry.

The following examples are provided to illustrate the present invention and are not to be construed as limiting the scope of the invention in any way.

## Example 1. Fermentation of Streptomyces griseorubiginosus S980-14

A loopful of Streptomyces griseorubiginosus S980-14 grown on agar slant (composed of soluble starch 0.5%, glucose 0.5%, fish meat extract 0.1%, yeast extract 0.1%, NZ-case 0.2%, NaCl 0.2%, $CaCO_3$ 0.1%, and agar 1.6%, pH 7.2) was transferred into liquid medium (100 ml of medium in 500 ml Erlenmeyer flask) composed of glucose 1% and yeast extract 1% (pH 7.2) and incubated for 4 days at 28°C on a rotary shaker set at 200 rpm. A 1 ml aliquot of the culture was inoculated into a 500 ml Erlenmeyer flask containing 100 ml of a medium composed of D-xylose 1% and yeast extract 1%, pH 7.2, and incubated for 4 days at 28°C on a rotary shaker set at 200 rpm.

## Example 2. Isolation and Purification of Arylsulfatase

Fermentation broth from 50 flasks was filtered to yield 4.8 L of filtrate (Stage 1) which was concentrated to 460 ml by ultrafiltration at room temperature using an ultrafiltration module (Asahi Kasei Co.) having a nominal molecular weight cutoff of 6,000 (Stage 2). Ammonium sulfate was added to this concentrated protein solution to achieve 30% (w/v) saturation, and the solution was allowed to stand at 4°C overnight. The solution was centrifuged at 13,000 rpm for 15 minutes at 4°C, and the supernate was applied to a column (1.5 x 4.5 cm) of Butyl-Toyopearl 650 C (TOSHO Co.) equilibrated with 30% ammonium sulfate in 50 mM Tris-HCl, pH 7.5. The resin was washed with 30% ammonium sulfate in the buffer, and Es-1 sulfatase was eluted with 50 mM Tris-HCl, pH 7.5 without ammonium sulfate. The eluate was collected in 5-ml fractions. The active fractions were combined (28 ml, Stage 3). The combined solution was applied to a column (1.5 x 4 cm) of DEAE-Cellulose (Seikagaku Kogyo Co.) equilibrated with 50 mM Tris-HCl, pH 7.5. The column was developed with a linear concentration gradient of Tris-HCl, pH 7.5, from 50 mM to 300 mM. The eluate was collected in 5-ml fractions to give 252 ml of the enzyme solution (Stage 4). The combined solution was mixed with ammonium sulfate at 30 (w/v)% saturation and applied on a column (1.5 x 3 cm) of Butyl-Toyopearl 650 M equilibrated with 30% ammonium sulfate in 50 mM Tris-HCl, pH 7.5. The chromatographic eluate was changed to the linear concentration gradient of ammonium slufate from 30% to 10%. The activity was eluted around 20% saturation of ammonium sulfate. Specific activity of Es-1 sulfatase significantly increased at this stage (Stage 5). A column (1.5 x 5 cm) of polybuffer exchanger 94 (Mono PHR 5/20, Pharmacia LKB Biotechnology) was equilibrated with 25 mM imidazole buffer, pH 7.4. The enzyme solution from Stage 5 was put on the column and eluted with polybuffer 74 (Pharmacia LKB Biotechnology) which had been adjusted to pH 4.0 with HCl. The eluate was collected in 5-ml fractions. The activity was eluted around pH 5.0 (Stage 6). Finally, the enzyme was further purified on a column (2.5 x 75 cm) of cellulofine GC-700-m (Seikagaku Kogyo Co.) by eluting with 250 mM Tris-HCl, pH 7.5. The eluate was collected in 2.5 ml fractions (Fig. 5). Fifty µl each fraction was, as usual, assayed for the activity. Fractions 84 to 103 were combined and concentrated with a UF module UHP-43 (Advantec Co.). The SDS-PAGE pattern of Es-1 sulfatase showed a single protein band at this stage (Stage 7).

The result after each purification step is provided in the following chart:

| Stage | Total Volume (ml) | Total Protein (mg) | Total Sulfatase Activity (U) | Total Specific Activity (U/mg) | Fold Increase | Recovery (%) |
|---|---|---|---|---|---|---|
| 1. Crude | 4,800 | 3,110 | 4,420 | 1.42 | 1 | 100 |
| 2. UF-Module | 460 | 2,640 | 3,934 | 1.49 | 1.05 | 89 |
| 3. 1st Butyl-Toyopearl | 28 | 217.6 | 2,329.6 | 10.7 | 7.54 | 52.7 |
| 4. DEAE-Cellulose | 252 | 42 | 1,136 | 27.0 | 19.0 | 25.7 |
| 5. 2nd Butyl-Toyopearl | 14 | 2.9 | 375.6 | 129.5 | 91.2 | 8.5 |
| 6. Chromatofocusing | 4.5 | 0.39 | 269.8 | 691.8 | 487.2 | 6.1 |
| 7. Cellulofine GC 700-m | 1.5 | 0.17 | 194.6 | 1,144.7 | 806.1 | 4.4 |

## Claims

1. A substantially purified sulfatase capable of catalyzing the conversion of a 4'-demethylepipodophyllotoxin glucoside 4'-sulfate into a 4'-demethylepipodophyllotoxin glucoside, said sulfatase having a molecular weight of about 45 kD and an isoelectric point of about 4.95.

2. A sulfatase of Claim 1 isolated from Streptomyces griseorubiginosus S980-14, or a variant thereof, or a mutant thereof.

3. A substantially purified sulfatase isolated from Streptomyces griseorubiginosus S980-14, said sulfatase being capable of catalyzing the conversion of etoposide 4'-sulfate into etoposide and characterized by having a molecular weight of about 45 kD and an isoelectric point of about 4.95.

4. A substantially purified sulfatase isolated from Streptomyces griseorubiginosus S980-14, said sulfatase being capable of catalyzing the conversion of 2",3"-di-O-acetyl-etoposide 4'-sulfate into 2",3"-di-O-acetyl-etoposide and is characterized by having a molecular weight of about 45 kD and an isoelectric point of about 4.95.

5. A method for the production of the sulfatase of Claim 1 which comprises cultivating Streptomyces griseorubiginosus S980-14, or a variant thereof, or a mutant thereof under submerged aerobic conditions in a medium containing assimilable sources of carbon and nitrogen until a recoverable amount of the sulfatase has been produced, and recovering said sulfatase.

6. A biologically pure culture of Streptomyces griseorubiginosus S980-14 having the identifying characteristics of ATCC 55031.

Fig. 1 Optimum pH (A) and pH stability (B) of Es-1 sulfatase

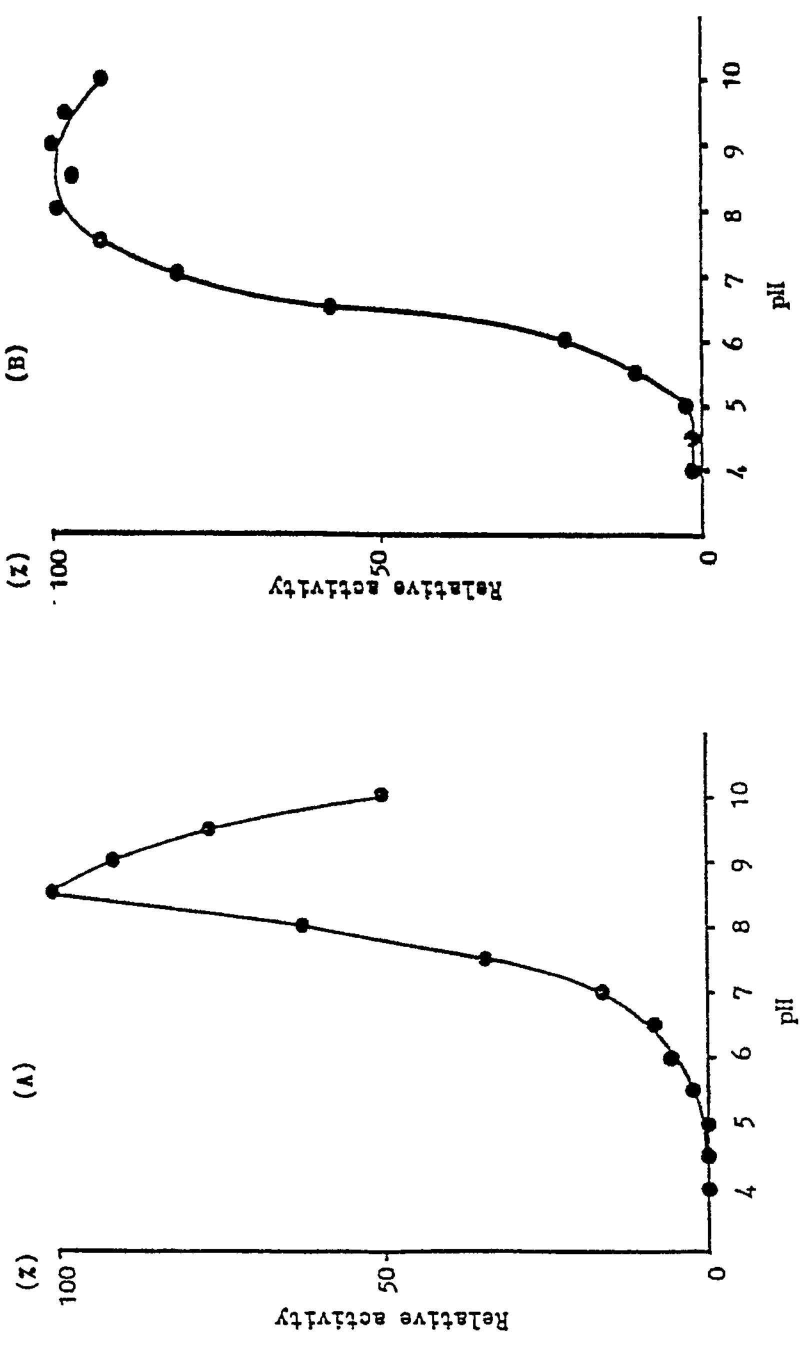

Fig. 2 Optimum temperature (A) and temperature stability (B) of Es-1 sulfatase

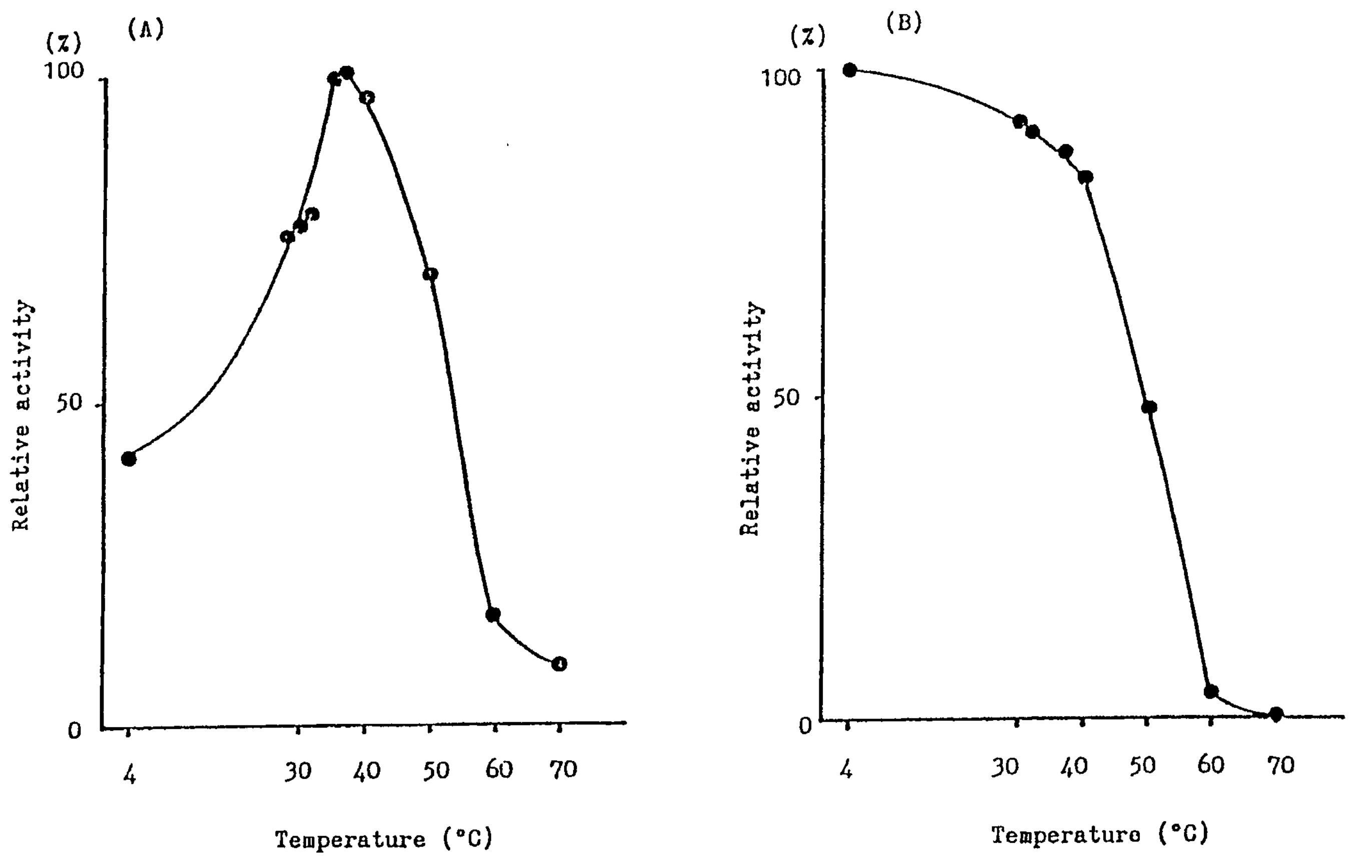

Fig. 3 Effect of $Ca^{2+}$ concentrations on EDTA-treated Es-1 sulfatase